# EUROPEAN PATENT SPECIFICATION

(11) **EP 2 379 307 B1**
(45) Date of publication and mention of the grant of the patent: **13.03.2013**
(21) Application number: 09796567.7
(22) Date of filing: 22.12.2009
(51) Int. Cl.: B29C 59/04, A61L 15/26, C08J 5/18, A44B 18/00, B29C 55/00, B32B 27/36

(54) **POLYLACTIDE FILMS HAVING STRUCTURED SURFACE AND METHODS FOR MAKING THE SAME**
POLYLAKTIDFOLIE MIT EINER STRUKTURIERTEN OBERFLÄCHE UND HERSTELLUNGSVERFAHREN DAFÜR
FILMS DE POLYLACTIDE AYANT UNE SURFACE STRUCTURÉE, ET PROCÉDÉS POUR LEUR RÉALISATION

(30) Priority: 29.12.2008 US 141120 P
(43) Date of publication of application: 26.10.2011
(73) Proprietor: 3M Innovative Properties Company, Saint Paul, MN 55133-3427 (US)
(72) Inventor: AUSEN, Ronald, W., Saint Paul, Minnesota 55133-3427 (US); BANY, Stephen, W., Saint Paul, Minnesota 55133-3427 (US); FRETAG, Joan, C., Saint Paul, Minnesota 55133-3427 (US); JENNEN, Jay, M., Saint Paul, Minnesota 55133-3427 (US); JOSEPH, Eugene, G., Blacksburg Virginia 24060 (US); MYERS, Sasha, B., Saint Paul, Minnesota 55133-3427 (US); WANG, Shou-Lu G., Saint Paul, Minnesota 55133-3427 (US)
(74) Representative: Vossius & Partner
(86) International application number: PCT/US2009/069108
(87) International publication number: WO 2010/078134

(56) References cited:
- WO-A1-03/016015
- WO-A2-2008/057214
- DE-A1- 19 829 936
- DE-U1-202006 001 693
- JP-A- 2000 136 357
- US-A- 5 930 876
- US-A1- 2005 112 350
- US-A1- 2005 206 048

## Description

### Background

Global plastic usage is estimated at 350 billion pounds per year. This success of using plastics has generated an issue due to its impact on the global infrastructure. As a result, useful articles containing renewable, degradable polymers are of significant interest. Renewable polymers are defined as those that are derived from natural or biomass materials. The use of renewable, degradable polymers to replace petroleum-based polymers is driven by a number of issues, including waste management, availability, and cost.

The use of plastics in disposable diapers and home care products constitutes a significant portion of plastics consumption in the global market. Thus, there is a need for renewable, degradable polymer films in such products. For example, such applications include renewable polymer hook and loop fasteners as the closure mechanism in diapers. Other applications include the use of renewable polymer matte-finished films in diapers and home care applications such as lint removal tapes.

Economic production of such films with appropriate physical properties is a challenge. For example, the polymer for hooks of hook and loop fasteners needs to have high modulus and toughness attributes for repeated anchorage to loops. They also need to have a certain amount of crystallinity for temperature and age stability. Such polymers need to be of relatively low cost, capable of crystallizing rapidly, capable of being formed into,hook stems and releasing from a tool roll at a high rate of speed, and in some cases capable of being subsequently formed into hooks in a capping operation. Similar characteristics are desired for embossed or imprinted matte-finished films. Thus, for example, hooks for hook and loop fasteners are typically produced from polypropylene, at a high rate of speed. A method for the production of separable fasteners that are made of a biodegradable resin, for example polylactic acid, is disclosed in US 5,930,876.

The first commercially available, relatively low cost, renewable, and degradable polymer is that produced from the polymerization of lactic acid or lactide. Bacterial fermentation is used to produce lactic acid from corn starch or cane sugar; however, lactic acid cannot be directly polymerized to a useful product, because each polymerization reaction generates one molecule of water, the presence of which degrades the forming polymer chain to the point that only very low molecular weights are observed. Thus, lactic acid is typically converted to the cyclic lactide monomer, which can be more readily polymerized into polymers having a wide range of molecular weights. Such material is typically referred to as "polylactic acid" polymer, or "polylactide" polymer, or "PLA." PLA has high surface energy, and films produced therefrom are smooth with a high gloss, shiny, and plastic-like surface finish. Typical matte-finished films produced from amorphous PLA do not retain the surface structure(s) and become smooth and shiny when heated above its glass transition temperature (Tg) (approximately 58°C).
Some examples of thermoforming of polylactid acid are disclosed in WO 03/016015 A1, WO 2008/057214 A2, DE 202006 001 693 U1 and DE 198 29 936 A1.

One problem of processing PLA rapidly into a continuous film is that it crystallizes very slowly. The addition of plasticizers and nucleating agents is known to increase the rate of crystallization; however, in a typical continuous film production process, when PLA is extruded onto a quenching tool roll above 65°C it sticks to the quenching chill roll. Thus, it is recommended that films be produced using a quenching temperature of 65°C or less. This makes it very difficult to form structured films with structures that are retained when subsequently heated.

For example, JP 2007-130893 demonstrates forming unstructured PLA films at lower casting temperatures but relies on two rolls in sequence wherein the first roll cools or quenches the material (to form crystalline nucleating material) and the second roll reheats the material to an annealing temperature. In this process, even if a matte-finish were disclosed as being applied by the first roll, it would subsequently anneal to a smooth surface upon reheating on the second roll.

Also, cold quenching temperatures do not allow the polymer to flow into the hook stem mold to form an adequate stem length. The cold quenching also prevents sufficient crystallization of the hook stem, which is important for subsequently being formed into hooks in a capping operation, and for thermal and age stability.

Thus, there is a need for embossed, structured films containing PLA produced in a rapid, cost effective process.

### Summary

The present invention provides films and methods of making films that include crystalline polylactide. Such films have an embossed, structured surface. Significantly, the structure(s) of the embossed, structured surface are retained (e.g., in size and shape) upon heating the film at a temperature of up to 130°C.

In one embodiment, the present invention provides a process for forming a film, the process comprising: applying a molten composition comprising polylactide to a tool roll having a structured surface, wherein the tool roll is at a temperature above the Tg and below the Tm of the polylactide-containing composition; allowing the molten composition to remain in contact with the tool roll for a time sufficient to convert at least a portion of the polylactide to crystalline polylactide; and removing a film comprising crystalline polylactide from the tool roll; wherein the film is continuous and has an embossed, structured surface comprising structure(s) in the form of a negative imprint of the tool roll structured surface; and further wherein the structure(s) of the embossed, structured surface are retained upon heating the film at a temperature of up to 130°C.

In certain embodiments, the polylactide has at least 1 wt-% crystallinity. In certain embodiments, the polylactide has no greater than 40 wt-% crystallinity. This crystallinity contributes to the ability of the structure(s) of the embossed, structured surface to be retained during, for example, transportation and storage. Typically, the size and shape of the structure(s) are retained upon heating the film at a temperature of up to 130°C.

A film of the present invention may have one structure (e.g., a continuous pattern) or a plurality of structures on the structured surface. For example, such structures may be in the form of hooks or stems. Such films can be formed in certain embodiments of the present invention by using a tool roll that has a structured surface comprising a plurality of mold cavities. A molten PLA-containing composition is applied to the tool roll under conditions effective to fill the mold cavities. Such conditions can include sufficient pressure (which can be readily determined by one of skill in the art), and a tool roll temperature of 100°C to 130°C. In certain embodiments, the mold cavities in the tool roll structured surface form upstanding hook stems on the surface of the film, and, if desired, such stems can be converted to headed stem mechanical fasteners.

Alternatively, a film of the present invention may have a matte finish. Such films can be formed in certain embodiments of the present invention by using a tool roll that has a structured surface that is textured. Preferably, the structured surfaces of such matte-finished films have an Ra of at least 1.25 microns. A molten PLA-containing composition is applied to the tool roll under conditions effective to transfer the texture of the structured tool roll surface to the film and provide a matte finish. Such conditions can include sufficient pressure (which can be readily determined by one of skill in the art), and a tool roll temperature of 85°C to 130°C.

In preferred embodiments of the process, the polylactide-containing composition includes a plasticizer. Typically, a plasticizer is selected that lowers the Tg of the polylactide-containing composition by greater than 5°C. In certain embodiments, the polylactide-containing composition comprises a plasticizer in an amount of at least 5 wt-%, based on the total weight of the PLA-containing composition.

In preferred embodiments of the process, the polylactide-containing composition includes a nucleating agent. The nucleating agent can be an inorganic nucleating agent (e.g., having an average particle size of 25 nanometers to 10 microns). Alternatively, the nucleating agent can be an organic polymeric nucleating agent.

The present invention also includes films made by any one of the processes described herein.

In one embodiment, the present invention provides a film comprising polylactide having at least 1 wt-% crystallinity, wherein the film is continuous and comprises an embossed, structured surface (one or both surfaces of the film can be structured) comprising structure(s) in the form of a negative imprint of a tool roll structured surface, wherein the structure(s) are retained upon heating the film at a temperature of up to 130°C. In certain embodiments, the polylactide has no greater than 40 wt-% crystallinity. This crystallinity contributes to the ability of the structure(s) of the embossed, structured surface to be retained during, for example, transportation and storage. The films of the present invention will typically include one or more plasticizers and one or more nucleating agents.

The films of the present invention can include a surface having a layer of adhesive thereon, and form, for example, a tape. Other products the films of the present invention can be used in include, for example, hook and loop fastener systems, which may be used in diapers. Other products include lint removal tapes and rollers, and laminates in which films of the present invention are laminated to other substrates such as nonwovens and paper.

Thus, the present invention provides, for example, a disposable garment such as a diaper comprising a film of the present invention. Such film can be a portion of a hook and loop fastener system, e.g., the hook portion, or it can be a backsheet of a diaper, for example.

A hook and loop fastener is also provided that includes a film comprising polylactide having at least 1 wt% crystallinity, wherein the film is continuous and comprises an embossed, structured surface comprising structure(s) in the form of a negative imprint of a tool roll structured surface, wherein the structure(s) are retained upon heating the film at a temperature of up to 130°C, and further wherein the structured surface comprises a plurality of hooks.

The present invention also provides a tape comprising a film having at least one surface with a layer of adhesive disposed thereon, wherein the film comprises polylactide having at least 1 wt-% crystallinity, and wherein the film is continuous and comprises an embossed, structured surface comprising structure(s) in the form of a negative imprint of a tool roll structured surface, wherein the structure(s) are retained upon heating the film at a temperature of up to 130°C, and further wherein the structured surface comprises a matte finish. The adhesive can be coated on the matte-finished surface or it can be coated on the (typically, smooth) surface opposite the matte-finished surface. Such tape can be in the form of a tape flag or it can be the tape used in a lint removal roller.

The terms "comprises" and variations thereof do not have a limiting meaning where these terms appear in the description and claims.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, "a," "an," "the," "at least one," and "one or more" are used interchangeably. Thus, for example, a composition comprising "a" nucleating agent can be interpreted to mean that the composition includes "one or more" nucleating agents. Similarly, a composition comprising "a" plasticizer can be interpreted to mean that the composition includes "one or more" plasticizers.

As used herein, the term "or" is generally employed in its sense including "and/or" unless the content clearly dictates otherwise. The term "and/or" means one or all of the listed elements or a combination of any two or more of the listed elements.

As used herein, all numbers are assumed to be modified by the term "about" and preferably by the term "exactly." Notwithstanding that the numerical ranges and parameters setting forth the broad scope of the invention are approximations, the numerical values set forth in the specific examples are reported as precisely as possible. All numerical values, however, inherently contain certain errors necessarily resulting from the standard deviation found in their respective testing measurements.

Also herein, the recitations of numerical ranges by endpoints include all numbers subsumed within that range (e.g., 1 to 5 includes 1, 1.5, 2, 2.75, 3, 3.80, 4, 5, etc.). Also, a numerical range that includes "up to" a certain value includes that value.

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### Brief Description of the Figures

FIG. 1 is partial schematic of a tool roll having a structured surface and a film formed thereon.

FIG. 2 is an enlarged perspective view of a stemmed web.

FIG. 3 is an enlarged perspective view of a hook fastener.

FIG. 4 is a perspective view of a disposable diaper using a hook fastener according to the present invention.

FIG. 5 is a perspective view of a roll of lint removal tape.

### Detailed Description of Illustrative Embodiments

The present invention provides films and methods of making films that include crystalline polylactide. Polylactide is a polymeric material that offers unique advantages as a film in the biodegradable sense. Although polylactide-containing films with increased crystallinity generally degrade more slowly than amorphous films under conditions of high humidity and heat, crystallinity contributes to temperature and age stability, as well as other properties desirable for structured films (i.e., films having a structured surface).

Lactic acid has two optical isomers, L-lactic acid, also known as (S)-lactic acid, and D-lactic acid, also known as (R)-lactic acid. Due to the chiral nature of lactic acid, several distinct forms of polylactide exist: L,L-lactide, also known as L-lactide, which comprises two (S)-lactic acid residuals; D,D-lactide, also known as D-lactide, which comprises two (R)-lactic acid residuals; and meso-lactide, which comprises one each of (R)- and (S)-lactic acid residuals. Polymerization of a racemic mixture of L- and D-lactides usually leads to the synthesis of poly-DL-lactide, which is not crystalline but amorphous. Although using certain catalysts during polymerization and/or controlling the ratio of D to L enantiomers can influence the crystallization kinetics of PLA, additives (e.g., nucleating agents) and processing parameters impact the level of crystallinity and the rate of crystallization.

In a method of the present invention, a process for forming a film is provided that results in a useable continuous film having suitable crystallinity for various applications. The process includes: applying a molten composition comprising (amorphous) polylactide to a tool roll having a structured surface, wherein the tool roll is at a temperature above the Tg and below the Tm of the polylactide-containing composition; allowing the molten composition to remain in contact with the tool roll for a time sufficient to convert at least a portion of the (amorphous) polylactide to crystalline polylactide; and removing a film comprising crystalline polylactide from the tool roll; wherein the film is continuous and has an embossed, structured surface comprising structure(s) in the form of a negative imprint of the tool roll structured surface; and further wherein the structure(s) of the embossed, structured surface is retained upon heating the film at a temperature of up to 130°C (although higher temperatures may be possible).

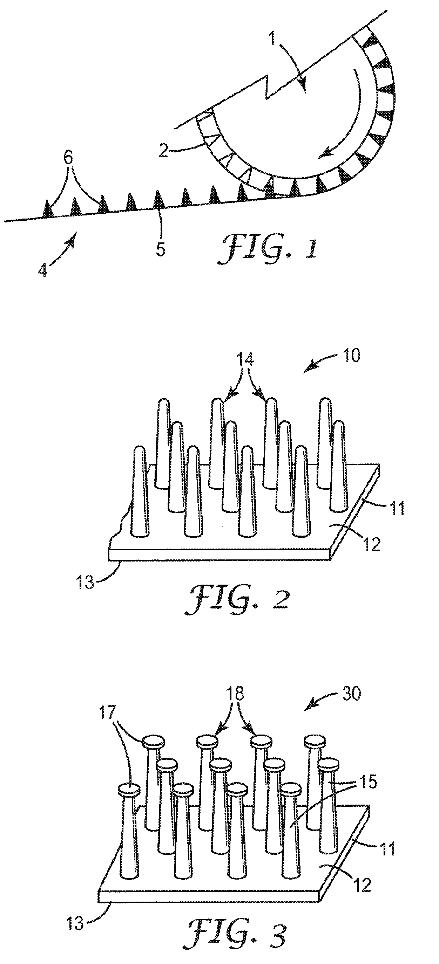

As shown in FIG. 1, which is a partial schematic of a tool roll 1 having a structured surface 2 (which can result, for example, from a structured belt, sleeve, wire, or the like), with a PLA-containing composition in contact with the tool roll, which forms a continuous film 4 that has an embossed, structured surface 5 comprising structure(s) 6 in the form of a negative imprint of the tool roll structured surface 2.

The resultant films are "continuous," which refers to a film that has an indefinite length that is much longer that it is wide (e.g., the length is at least 5 times the width, at least 10 times the width, or at least 15 times the width). Such continuous films have an embossed, structured surface with structure(s) in the form of a negative imprint of the tool roll structured surface.

Significantly, such structure(s) of the embossed, structured surface are retained upon heating the film at a temperature of up to 130°C. Such retention is due, at least in part, to the presence of crystallinity in the PLA. Thus, the process of forming the films of the present invention provides films that are generally stable during storage and transportation.

Desirable levels of crystallinity for films of the present invention, on a weight basis, are preferably at least 1 percent by weight (wt-%), more preferably at least 2 wt-%, even more preferably at least 5 wt-%, and even more preferably at least 10 wt-%. Typically, and preferably, films of the present invention have no greater than 40 wt-% crystallinity.

The structure(s) on the structured surface of the tool roll can be in the form of one continuous structure or pattern (e.g., a cross-hatched pattern) or multiple structures (e.g., cavities for forming hook stems). Structures are embossed into the PLA film as a result of transferring an imprint of the tool roll structured surface to the film. Such tool roll structured surfaces can include random structures. Alternatively, it can have a more structured machine finish. For example, the tool roll structured surface can be textured for providing a matte surface to the film. If desired, both surfaces of the films provided herein can have structures imprinted therein.

In certain embodiments, the tool roll structured surface comprises a plurality of mold cavities. Such mold cavities can have a suitable shape that results in forming upstanding hook stems, or hook-like projections, for example, on the surface of the film. In methods using such tool roll, the molten composition is applied to the tool roll under conditions effective to fill the mold cavities. Such conditions, including time and temperature, would be readily determined by one of skill in the art based on the teachings herein.

In the process of the present invention, the tool roll is at a temperature above the Tg of the polylactide-containing composition and below the Tm of the polylactide-containing composition. Although the Tg of a typical commercially available PLA is approximately 58°C, this can vary depending on the formulation of the polylactide-containing composition. It can be as low as 30°C (Tg). In certain preferred embodiments, the tool roll is at a temperature of at least 85°C, at least 100°C, above 100°C, or at least 105°C. Similarly, although the melting temperature (Tm) of a typical commercially available PLA is approximately 160°C, this can vary depending on the formulation of the polylactide-containing composition. Thus, in certain preferred embodiments, the tool roll is at a temperature of no greater than 130°C.

Such temperatures can enhance the rate of crystallization of the polylactide, enhance the filling of mold cavities, and reduce sticking of the film to the tool roll thereby enhancing processing speeds, for example. A particular exemplary process uses a tool roll at a temperature of 85°C to 130°C. Another exemplary process uses a tool roll at a temperature of 100°C to 130°C. Another exemplary process uses a tool roll at a temperature of 105°C to 130°C.

Sufficient pressure applied to the molten composition in the area where it contacts the tool roll (often referred to as the nipped area) can be determined by one of skill in the art. Such pressure can affect the formation of the initial structures in the surface. Typically, the higher the temperature of the tool roll, the lower the pressure that may be required.

Equipment set-ups for preparing continuous films of the present invention are well-known to those of skill in the art. Exemplary equipment set-ups are described in the Examples Section. For example, a typical tool roll is made of steel, although other materials of the tool roll can include nickel- or chrome-plated steel.

Typically, a process of the present invention uses one major tool roll. For example, although other rolls may be used (such as a nip or back-up rubber or steel roll), a typical process does not include two rolls in sequence to cool and subsequently reheat the temperature of the composition or film, as is done in JP 2007-130893. Thus, a preferred process of the present invention of forming a film is done in one step, e.g., one major structure-forming step.

In certain embodiments, the present invention also provides a process of forming a headed stem mechanical fastener out of the upstanding stems on the surface of the film. FIG. 2 is an enlarged perspective view of a stemmed web that can be used to make a hook fastener (also referred to as a headed stem mechanical fastener), as shown in FIG. 3. This process results in a film with an array of upstanding hook elements with upstanding stem base portions and hook heads or head portions with outward projecting (and sometimes downwardly projecting) fiber engaging portions. Processes for making headed stem mechanical fasteners are described in, for example, U.S. Pat. Nos. 6,132,660, 6,039,911, 5,679,302, and 6,635,212.

Referring now to FIG. 2, an exemplary stemmed web, which can be produced according to the present invention is generally designated by the reference numeral 10. The stemmed web 10 comprises a thin strong flexible film-like backing 11 having generally parallel upper and lower major surfaces 12 and 13, and a multiplicity of spaced stems 14 projecting from at least the upper surface 12 of the backing 11. The backing can have planar surfaces or surface features as could be desired for tear resistance or reinforcement.

Such stemmed web can be converted to a hook fastener (also referred to as a headed stem mechanical fastener) 30, as shown in FIG. 3, wherein the hook elements (i.e., hooks) 18 each comprise a stem portion 15 attached at one end to the backing 11 (having generally parallel upper and lower major surfaces 12 and 13) and preferably having tapered sections that widen toward the backing 11 to increase the hook anchorage and breaking strengths at their junctures with the backing 11, and a head portion 17 at the end of the stem portion 15 opposite the backing 11.

The geometrical shape of the hook heads is not particularly limited and comprises a wide variety of shapes, as described in the literature. The stem portions may have an essentially circular cross-section but other shapes such as, for example, essentially triangular or rectangular cross-sections or less regular cross-sections are also possible. The thickness of the stem portions along its vertical extension from the backing 11 to the hook head portion 17 (FIG. 3) may be essentially constant but may also vary whereby an essentially constant thickness or a thickness essentially decreasing from the backing 11 to the hook head portion 17, are preferred.

The size of the hook elements can vary over a broad range. The present hook elements (also known as headed stem fasteners) are particularly useful on low-cost, disposable items such as diapers. For use on diapers, the hooks are of uniform height, preferably of from 0.1 millimeters (mm) to 1.3 mm in height, and more preferably from 0.2 mm to 0.5 mm in height. The hooks have a density on the backing preferably of from 60 to 1,600 hooks per square centimeter (cm²), and more preferably from about 125 to 700 hooks per square centimeter. The stems have a diameter adjacent the heads of the hooks preferably from 0.1 mm to 0.6 mm, and more preferably from 0.1 mm to 0.3 mm. The heads that project radially past the stems on each side preferably by an average of 0.01 mm to 0.25 mm, and more preferably by an average of 0.025 m to 0.13 mm and have average thicknesses between their outer and inner surfaces (i.e., measured in a direction parallel to the axis of the stems) preferably of from 0.01 mm to 0.25 mm and more preferably of from 0.025 mm to 0.13 mm. The heads have an average diameter (i.e., measured radially of the axis of the heads and stems) to average head thickness ratio preferably of from 1.5:1 to 12:1, and more preferably from 2.5:1 to 6:1.

Another embodiment of the present invention is a matte-finished film. The structures of an exemplary matte-finished film structured surface preferably have an Ra of at least 1.25 microns. Roughness average (Ra) is the arithmetic sampling average of the absolute values of the measured profile height deviations measured from the centerline. These structures are typically and preferably retained, preferably at this Ra value, upon heating the film to a temperature up to (and including) 100°C.

The PLA-containing composition contains PLA, and optionally, other polymers compatible with PLA. Preferably, the polylactide includes less than 5 wt-% d-lactide, or less than 2 wt-% d-lactide. Preferably, the polylactide includes no more than 20 wt-% d-lactide.

An exemplary commercially available PLA resin suitable for extrusion or thermoforming is available under the trade designation NatureWorks PLA polymer 2002D, 4032D, and 4042D from NatureWorks, Minnetonka, MN. Other commercially available PLA resins include film-grade, sheet-grade, nonwoven-grade, or injection molding-grade materials.

PLA-containing compositions of the present invention preferably include one or more plasticizers and/or one or more nucleating agents. Typically, such plasticizers and/or nucleating agents are retained in the resultant films.

Plasticizers can improve the film properties of the lactide polymer (e.g., flexibility, impact, tear resistance), although they are typically used to reduce the melt viscosity at a given temperature of the composition, which assists in processing and extruding the polymer at lower temperatures. Exemplary plasticizers lower the Tg of the polylactide-containing composition by greater than 5°C, and preferably by 20-30°C.

Desirably, a plasticizing agent (i.e., plasticizer) for use in a biodegradable film should itself be biodegradable and compatible with the resin. Exemplary plasticizers may also be relatively nonvolatile. Suitable plasticizing agents are disclosed, for example, in U.S. Pat. No. 6,121,410, JP 2007-130893, and U.S. Pat. Publ. 2007/0160782.

Examples of suitable plasticizers are in the general classes of alkyl or aliphatic esters, ethers, and multi-functional esters and/or ethers. These include alkyl phosphate esters, dialkylether diesters, tricarboxylic esters, epoxidized oils and esters, polyesters, polyglycol diesters, alkyl alkylether diesters, aliphatic diesters, alkylether monoesters, citrate esters, dicarboxylic esters, vegetable oils and their derivatives, and esters of glycerine. Most preferred plasticizers are tricarboxylic esters, citrate esters, esters of glycerine and dicarboxylic esters. Plasticizers containing aromatic functionality or halogens are not preferred because of their possible negative impact on the environment.

For example, appropriate character is exhibited by triethyl citrate, acetyl triethyl citrate, tri-n-butyl citrate, acetyl tri-n-butyl citrate, acetyl tri-n-hexyl citrate, n-butyl tri-n-hexyl citrate and dioctyl adipate. Appropriate compatibility is exhibited by acetyl tri-n-butyl citrate and dioctyl adipate. Other compatible plasticizers include any plasticizers or combination of plasticizers which can be blended with polylactide and are either miscible with polylactide or which form a mechanically stable blend.

Volatility is determined by the vapor pressure of the plasticizer. An appropriate plasticizer is sufficiently nonvolatile such that the plasticizer stays substantially in the resin formulation throughout the process needed to produce the film. Excessive volatility can lead to fouling of process equipment, which is observed when producing films by melt processing polylactide with a high lactide content. Preferred plasticizers have a vapor pressure of less than about 10 mm Hg at 170°C, more preferred plasticizers have a vapor pressure of less than 10 mm Hg at 200°C.

Internal plasticizers, which are bonded to the polylactide, may also be useful. Epoxides provide one method of introducing an internal plasticizer.

A plasticizer is useful at levels of at least 5 wt-%, based on the total weight of the PLA-containing composition. Preferably, a plasticizer is used at a level of at least 10 wt-%, and more preferably at least 15 wt-%, based on the total weight of the PLA-containing composition. A plasticizer is useful at levels of no greater than 30 wt-%, based on the total weight of the PLA-containing composition. Preferably, a plasticizer is used at a level of no greater than 20 wt%, based on the total weight of the PLA-containing composition.

Nucleating agents are typically used to provide a heterogeneous surface on which crystallization can begin. They can be inorganic or organic materials. Suitable nucleating agents are disclosed, for example, in U.S. Pat. No. 6,121,410, JP 2007-130893, and U.S. Pat. Publ. 2007/0160782. Nucleating agents may include selected plasticizers, finely divided minerals, organic compounds, salts of organic acids and imides and finely divided crystalline polymers with a melting point above the processing temperature of the polylactide.

Preferred nucleating agents include inorganic nucleating agents and organic polymeric nucleating agents, and more preferably, inorganic nucleating agents. The small organic molecules described as suitable nucleating agents in JP 2007-130893 are not generally suitable for use in the present invention.

Examples of useful nucleating agents include, for example, talc, zinc oxide, sodium salt of saccharin, calcium silicate, sodium benzoate, calcium titanate, boron nitride, and copper phthalocyanine.

Examples of suitable inorganic nucleating agents have an average particle size of at least 25 nanometers, or at least 0.1 micron. Examples of suitable inorganic nucleating agents have an average particle size of no greater than 10 microns.

A nucleating agent is useful in levels of at least 0.1 wt-%, based on the total weight of the PLA-containing composition. Preferably, a nucleating agent is used at a level of at least 0.5 wt-%, more preferably at least 1.0 wt-%, and even more preferably at least 2.0 wt-%, based on the total weight of the PLA-containing composition. High levels of such nucleating agents may be used, if desired, as fillers.

A preferred film of the present invention is one that includes polylactide having at least 1 wt-% crystallinity, wherein the film is continuous and has an embossed, structured surface comprising structure(s) in the form of a negative imprint of a tool roll structured surface, wherein the structure(s) are retained upon heating the film at a temperature of up to 100°C. Such structures preferably have an Ra of at least 1.25 microns.

The films of the present invention are typically not oriented. That is they are typically not post-oriented, although they may be slightly oriented coming out of the die.

PLA-containing films of the present invention can be used in a variety of products. For example, they can be used as hook and loop fasteners in the closure mechanism on disposable garments, such as diapers or hospital gowns, as the backsheet of a diaper, in tape (such as diaper tape), tape flags, lint removal tapes (e.g., in lint rollers), and in laminates of the films to other substrates such as nonwovens and paper. For example, a matte-finished PLA-containing film can be used in diapers (e.g., as the backsheet or tape backing), tapes, tape flags, and home care applications such as lint removal tapes. The matte-finished surface can be on one side of a matte-finished film of the present invention or on both sides if desired. The adhesive in a tape that uses a matte-finished film of the present invention as a backing can be disposed on the matte-finished surface or on the opposite (typically, smooth) surface.

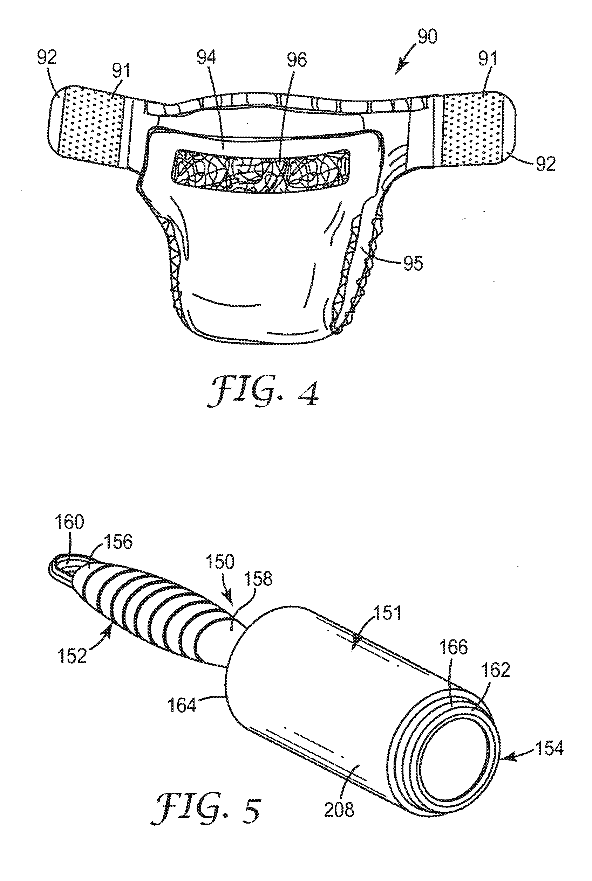

A conventional diaper construction is depicted in FIG. 4. The diaper 90 is provided with a thin liquid-impermeable outer backsheet 94 and a liquid-permeable inner coversheet 95. Between the backsheet 94 and inner coversheet 95 is an absorbent core (not shown). In FIG. 4, a large area hook tab 91 is attached to a carrier substrate 92, which is attached to a diaper 90 as is known in the art. Alternatively, the hook tab can be directly bonded to the diaper 95, either at an ear cutout portion or at the edge region of the diaper. In any of these embodiments, the hook tab 91 would engage a suitable mating region 96 on the backsheet 94. The backsheet 94 can be a thin polyethylene film, or it can be a matte-finished PLA-containing film of the present invention, while the inner coversheet 95 would typically be a nonwoven such as a spunbond polypropylene.

In place of a hook and loop fastener system, such disposable garments can include adhesive fastening tabs (e.g., diaper tapes). Such tapes can include a PLA-containing film, such as a matte-finished film, of the present invention comprising a surface having a layer of adhesive thereon. In addition to diaper tapes, tapes of the present invention could be used in a wide variety of other applications, such as tape flags or the tape used in a lint removal roller. The adhesive can be disposed on a matte-finished surface of such film or on the opposite (typically, smooth) surface. A wide variety of adhesives can be used as are known in the art. Exemplary adhesives are pressure-sensitive adhesives, such as a tackified elastomer where the elastomer is an A-B type block copolymer, wherein the A blocks and the B blocks are configured in linear, radial, or star configurations. A wide variety of other adhesives can be used as is well-known in the art.

Similarly, such adhesives and PLA-containing matte-finished films of the present invention can also be used in lint removal tapes, as used, for example, in lint rollers. FIG. 5 illustrates a roll 151 of lint removal tape 208, which includes adhesive coated on a matte-finished PLA-containing film of the present invention, on an applicator 150 to provide a lint removal tape assembly. A handle portion 152 can have any shape and can be contoured to ergonomically fit a hand. The handle portion 152 has a free end 156 and a connecting end 158. The free end 156 can have an opening 160 to permit hanging the applicator 150 on a hook for storage. A tape-receiving portion is shown at 154 and includes a free end 162 and a connecting end 164. The connecting end 158 of the handle portion 152 is connected to the connecting end 164 of the tape-receiving portion 154. The tape-receiving portion 154 also includes a cylindrical tape receiving surface 166, which extends between the free end 162 and the connecting end 164. The tape-receiving surface 166 extends for the entire width of a tape roll 151 and provides support along substantially the entire surface of the tape roll. The tape-receiving surface 166 is typically cylindrical, but it need not be. It could be formed of planar or curved sides meeting in edges that assist in holding the tape roll 151 in position.

Roll 151 may optionally include a core, where the multiple wraps of lint removal tape 208 would be wound about the core. Roll 151 may include an optional liner interposed between multiple wraps of tape 208. The lint removal tape 208 is illustrated as having the layer of adhesive coated across the entire width of the lint removal tape 208. Alternatively, the tape 208, which includes a PLA-containing film of the present invention, may include one or any number of non-adhesive zones. These non-adhesive zones would help the user separate the outer wrap of tape 208 from the roll 5. A first non-adhesive zone could run along the first edge of the length of the lint removal tape 208. A second non-adhesive zone could run along the second edge of the length of the lint removal tape 208, opposite the first non-adhesive zone. Both non-adhesive zones could run along the length of the lint removal tape 208 opposite each other with the layer of adhesive located in between. The non-adhesive zones could be first adhesive coated, along with the rest of the tape, and then detackified by using waxes, lacquers, or inks, for example. Alternatively, the first and second non-adhesive zones could be left uncoated-by adhesive.

PLA-containing films of the present invention can be used in a wide variety of other applications where degradable polymers are desired.

### -Exemplary Embodiments

1. A process for forming a film, the process comprising:
   applying a molten composition comprising polylactide to a tool roll having a structured surface, wherein the tool roll is at a temperature above the Tg and below the Tm of the polylactide-containing composition;
   allowing the molten composition to remain in contact with the tool roll for a time sufficient to convert at least a portion of the polylactide to crystalline polylactide; and
   removing a film comprising crystalline polylactide from the tool roll;
   wherein the film is continuous and has an embossed, structured surface comprising structure(s) in the form of a negative imprint of the tool roll structured surface;
   and further wherein the structure(s) of the embossed, structured surface are retained upon heating the film at a temperature of up to 130°C.
2. The process of embodiment 1 wherein the film comprises polylactide having at least 1 wt-% crystallinity.
3. The process of embodiment 1 or 2 wherein the film comprises polylactide having no greater than 40 wt-% crystallinity.
4. The process of any one of embodiments 1 through 3 wherein the tool roll structured surface comprises a plurality of mold cavities and the molten composition is applied to the tool roll under conditions effective to fill the mold cavities.
5. The process of embodiment 4 wherein the tool roll is at a temperature of 100°C to 130°C.
6. The process of embodiment 4 wherein mold cavities in the tool roll structured surface form upstanding hook stems on the surface of the film.
7. The process of embodiment 6 further comprising forming a headed stem mechanical fastener out of the upstanding hook stems on the surface of the film.
8. The process of any one of embodiments 1 through 3 wherein the tool roll structured surface is textured, and the molten composition is applied to the tool roll under conditions effective to transfer the texture of the structured tool roll surface to the film and provide a matte finish.
9. The process of embodiment 8 wherein the structures of the film surface have an Ra of at least 1.25 microns.
10. The process of embodiment 8 or 9 wherein the tool roll is at a temperature of 85°C to 130°C.
11. The process of any one of embodiments 1 through 3 wherein the tool roll is at a temperature of at least 85°C.
12. The process of embodiment 11 wherein the tool roll is at a temperature of at least 100°C.
13. The process of embodiment 12 wherein the tool roll is at a temperature of above 100°C.
14. The process of embodiment 13 wherein the tool roll is at a temperature of at least 105°C.
15. The process of any one of embodiments 1 through 3 wherein the tool roll is at a temperature of no greater than 130°C.
16. The process of any one of embodiments 1 through 15 with the proviso that the process does not include two rolls in sequence to cool and subsequently reheat the composition or film.
17. The process of any one of embodiments 1 through 16 wherein forming a film comprising crystalline polylactide is done in one step.
18. The process of any one of embodiments 1 through 17 wherein the polylactide-containing composition comprises a plasticizer.
19. The process of embodiment 18 wherein the polylactide-containing composition comprises a plasticizer that lowers the Tg of the polylactide-containing composition by greater than 5°C.
20. The process of embodiment 18 or 19 wherein the polylactide-containing composition comprises a plasticizer in an amount of at least 5 wt%, based on the total weight of the PLA-containing composition.
21. The process of any one of embodiments 1 through 20 wherein the polylactide-containing composition comprises a nucleating agent.
22. The process of embodiment 21 wherein the polylactide-containing composition comprises a nucleating agent in an amount of at least 0.1 wt-%, based on the total weight of the PLA-containing composition.
23. The process of embodiment 21 or 22 wherein the nucleating agent comprises an inorganic nucleating agent.
24. The process of embodiment 23 wherein the inorganic nucleating agent has an average particle size of 25 nanometers to 10 microns.
25. The process of embodiment 21 or 22 wherein the nucleating agent comprises an organic polymeric nucleating agent.
26. The process of any one of embodiments 1 through 25 wherein the polylactide comprises less than 5 wt-% d-lactide.
27. A film made by the process of any one of embodiments 1 through 26.
28. A film comprising polylactide having at least 1 wt% crystallinity, wherein the film is continuous and comprises an embossed, structured surface comprising structure(s) in the form of a negative imprint of a tool roll structured surface, wherein the structure(s) are retained upon heating the film at a temperature of up to 130°C.
29. The film of embodiment 28 wherein the polylactide comprises no greater than 40 wt-% crystallinity.
30. The film of embodiment 28 or 29 wherein the structured surface comprises a plurality of hooks or stems.
31. The film of embodiment 28 or 29 wherein the structured surface comprises a matte finish.
32. The film of embodiment 31 wherein the matte surface structure(s) have an Ra of at least 1.25 microns.
33. The film of any one of embodiments 28 through 32 further comprising a plasticizer.
34. The film of any one of embodiments 28 through 33 further comprising a nucleating agent.
35. The film of embodiment 34 wherein the nucleating agent comprises an inorganic nucleating agent.
36. The film of embodiment 35 wherein the inorganic nucleating agent has an average particle size of 25 nanometers to 10 microns.
37. The film of embodiment 34 wherein the nucleating agent comprises an organic polymeric nucleating agent.
38. The film of any one of embodiments 28 through 37 wherein the polylactide comprises less than 5 wt-% d-lactide.
39. The film of any one of embodiments 28 through 38 further comprising a surface having a layer of adhesive disposed thereon.
40. A disposable garment comprising the film of any one of embodiments 28 through 39.
41. The disposable garment of embodiment 40 which is a diaper.
42. A tape comprising a film having at least one surface with a layer of adhesive disposed thereon, wherein the film comprises polylactide having at least 1 wt% crystallinity, and wherein the film is continuous and comprises an embossed, structured surface comprising structure(s) in the form of a negative imprint of a tool roll structured surface, wherein the structure(s) are retained upon heating the film at a temperature of up to 130°C, and further wherein the structured surface comprises a matte finish.
43. The tape of embodiment 42 which is a tape flag.
44. A lint roller comprising the tape of embodiment 42.
45. A hook and loop fastener comprising a film comprising polylactide having at least 1 wt-% crystallinity, wherein the film is continuous and comprises an embossed, structured surface comprising structure(s) in the form of a negative imprint of a tool roll structured surface, wherein the structure(s) are retained upon heating the film at a temperature of up to 130°C, and further wherein the structured surface comprises a plurality of hooks.

### Examples

Objects and advantages of this invention are further illustrated by the following examples, but the particular materials and amounts thereof recited in these examples, as well as other conditions and details, should not be construed to unduly limit this invention.

### Example 1

A structured surface film having a matte finish on one side was prepared using a polylactic acid (PLA) polymer (4032D obtained from Natureworks LLC, Minnetonka, MN) and the following procedure. A 40-mm 10-zone twin screw extruder was used to melt and extrude the PLA polymer, plasticizer, and nucleating agent to a positive displacement metering pump and then into a 25-centimeter (25-cm) wide conventional coat-hanger film die. The PLA polymer was dried for a minimum of 12 hours at 60°C to remove any moisture and then fed to the first zone of the extruder using a loss-in-weight feeder at a feed rate of 4.54 kilograms per hour (kg/hr). The first zone was water-cooled at approximately 25°C. The second zone of the extruder was set at 210°C while the remaining eight zones were set at 180°C. The die temperature was maintained at 170°C. A nucleating agent (UltraTalc 609 talc, obtained from Specialty Minerals, Bethlehem, PA) was fed to the feed throat of the extruder using a loss-in-weight feeder at a rate to achieve a 2.5% by weight of talc based on the final extruded composition. The extruder speed was set at 200 revolutions per minute (RPM). An acetyl tri-n-butyl citrate plasticizer (CITROFLEX A-4, obtained from Vertellus Performance Materials, Greensboro, NC) was fed into zone 3 of the extruder using a gridmelter (Dynatec, Hendersonville, TN) at a feed rate of 14.6% by weight based on the final extruded composition. The extrudate from the extruder was deposited vertically downward into a nip consisting of a 48-cm diameter temperature controlled matte finish steel tool roll (102°C) on one side and a 20-cm diameter chill (cooling) roll on the opposite side. A nip force of 73 N per lineal cm was used. A continuous silicone rubber belt was wrapped around the cooling roll (approximately 180 degrees of wrap) to aid in the extrusion process. The inner surface (the surface not in contact with the extrudate) of the belt was cooled with two steel rolls at a setpoint of 16°C. The extrudate remained in contact with the belt and tool roll for approximately 180 degrees of the tool roll circumference measured from the point of initial extrudate deposition. The cooled extruded film was then separated from the belt, and remained in contact with the tool roll for an additional approximate 60 degrees of wrap before being wound into a continuous roll. The film was pulled from the tool roll at 4.6 meters/minute (m/min) using a driven peel-off rubber coated roll that was slightly oversped relative to the tool roll speed. The tool roll was prepared by sandblasting a chrome-plated steel roll to achieve an average Ra roughness of 5.9 microns. Film windup speed was adjusted to achieve a film thickness of approximately 65 microns.

### Example 2

A structured surface film was prepared as in Example 1 except polylactic acid resin 4042D obtained from Natureworks was used.

### Comparative Example C1

A structured surface film was prepared as in Example 2 except that the matte finish steel tool roll was maintained at a set point temperature of 35°C and a nip force of 146 N per lineal cm was used. The low tool roll temperature did not provide adequate crystallinity to the film to enable the film to retain the imparted matte finish after annealing (heat treating) even with a doubling of the nip force, as shown in Table 1 below.

### Example 3

A structured surface film was prepared as in Example 1 except that a PLA feed rate of 9.1 kg/hr was used. CITROFLEX B-6 (obtained from Vertellus Performance Materials, Greensboro, NC) was used as a plasticizer at a feed rate of 14.6% by weight based on the final extruded composition. Zone 2 of the extruder was set at 160°C, with the remaining eight zones set at 180°C. The die temperature was maintained at 180°C. A nip force of 97 N per lineal cm was used.

### Example 4

A structured surface film was prepared as in Example 1 except that a PLA feed rate of 18.2 kg/hr was used. Zone 2 of the extruder was set at 160°C, with the remaining eight zones set at 180°C. A nip force of 12 N per lineal cm was used. The film was pulled from the tool roll at 18.4 m/min.

### Example 5

A structured surface film was prepared as in Example 1 except that the CITROFLEX A-4 plasticizer was used at a feed rate of 15% by weight based on the final extruded composition and no nucleating agent was used. Zone 2 of the extruder was set at 160°C. The die temperature was maintained at 160°C. A nip force of 12 N per lineal cm was used. The film was pulled from the tool roll at 3.1 meters/min.

### Comparative Example C2

A structured surface film was prepared as in Example 5 except that an 18-mm twin screw extruder having 8 zones was used to extrude the PLA composition. UltraTalc 609 talc was used as a nucleating agent at 0.5% by weight based on the final extruded composition. The first zone of the extruder was water-cooled at approximately 25°C. Zones 2-10 of the extruder were set at 210°C. The die temperature was maintained at 180°C. The matte finish steel tool roll was maintained at a set point temperature of 45°C and a nip force of 194 N per lineal cm was used. The film was pulled from the tool roll at 4.6 m/min. The low tool roll temperature did not provide adequate crystallinity to the film to enable the film to retain the imparted matte finish after annealing (heat treating) even with an increased nip force, as shown in Table 1 below.

### Example 6

A structured surface film having an array of upstanding stems on one side was prepared using a polylactic acid (PLA) polymer (4032D obtained from Natureworks LLC, Minnetonka, MN) and the following procedure. A 40-mm 8-zone twin screw extruder was used to melt and extrude the PLA polymer, plasticizer, and nucleating agent to a positive displacement metering pump and then into a 46 centimeter wide conventional coat-hanger film die (obtained from Cloeren Co., Orange, TX). The PLA polymer was dried for a minimum of 12 hours at 60°C to remove any moisture and then fed to the first zone of the extruder using a loss-in-weight feeder at a feed rate of 19.5 kg/hr. The first zone of the extruder was water-cooled at approximately 25°C. The remaining seven zones were set at 210°C. The die temperature was maintained at 210°C. A nucleating agent (UltraTalc 609 talc, obtained from Specialty Minerals, Bethlehem, PA) was fed to the feed throat of the extruder using a loss-in-weight feeder at a rate to achieve a 2.5% by weight of talc based on the final extruded composition. The extruder speed was set at 150 RPM. An acetyl tri-n-butyl citrate plasticizer (Citroflex A-4, obtained from Vertellus Performance Materials, Greensboro, NC) was fed into zone 3 of the extruder using a gridmelter (Dynatec, Hendersonville, TN) at a feed rate of 13.5% by weight based on the final extruded composition. The extrudate from the extruder was deposited vertically downward into a fixed gap nip consisting of a 25-cm diameter temperature controlled chrome finish steel tool roll (100°C) on one side and a 30-cm diameter chill (cooling) roll on the opposite side. The gap was adjusted to achieve the desired stem height of 360 microns or greater. The tool roll was wound with a profiled wire as described in U.S. Pat. No. 6,902,389 to provide a tool roll having 360 stem holes/cm², a stem hole diameter of 127 microns, and a stem hole depth of 420 microns. A continuous silicone rubber belt was wrapped around the cooling roll to aid in the extrusion process. The extrudate remained in contact with the belt and tool roll for approximately 120 degrees of the tool roll circumference measured from the point of initial extrudate deposition. The cooled extruded film was then separated from the belt, and remained in contact with the tool roll for an additional approximate 90 degrees of wrap before being wound into a continuous roll. The film was pulled from the tool roll at 8 m/min using a driven peel-off rubber coated roll that was slightly oversped relative to the tool roll speed. Film windup speed was adjusted to achieve a base film thickness (not including the stems) of approximately 123 microns.

The resulting structured surface film having upstanding stems was then transformed into a film having hook-like structures using the capping procedure as described in U.S. Pat. No. 5,679,302. The structured surface film was run through a nip between two steel calendar rolls spaced apart by 283 microns with the stem side of the film facing upward. The upper steel roll was maintained at a temperature of 143°C and was operated at a surface speed of 7 m/min. The lower steel roll was maintained at a temperature of 135°C and was operated at a surface speed of 3.4 meters per minute. The structured surface film was then processed using the procedure described in U.S. Pat. No. 6,132,660. The upper (top) silicone rubber-covered steel roll was maintained at a temperature of 138°C. The durometer (hardness) of the rubber was 58 Shore A. The lower (bottom) steel roll was maintained at a temperature of 38°C. The film was drawn through the nip between the upper and lower rolls at a speed of 4 meters/minute. A mushroom-type hook strip was produced similar to that shown in FIG. 3.

### Example 7

A structured surface film was prepared as in Example 6 except that the film windup speed was 20 m/min.

### Comparative Example C3

A structured surface film was prepared as in Example 7 except that the tool roll temperature was maintained at 27°C. The low tool roll temperature resulted in poorly formed stems with low heights.

### Testing

The surface roughness of Examples 1-5 and Comparative Examples C1 and C2 were measured using a Perthometer MP4 (obtained from Mahr Corp., Cincinnati, OH) using a 10 micron diameter stylus with the cutoff set at 2.5 mm. The film samples were placed on a smooth glass plate having an Ra roughness of 0 micron for measurement. The film samples were measured initially for roughness and then were annealed (heat treated) for 1 minute at 100°C in an oven and then measured again for roughness in approximately the same region. Results are shown in Table 1 below.

The heights of the stems of Examples 6-7 and Comparative example C3 were measured with an optical measuring microscope with a resolution of +/- 5 microns. The film samples were measured initially for stem height and then were annealed (heat treated) for 1 minute at 100°C in an oven and then measured again for stem height in approximately the same region. Results are shown in Table 2 below.

The crystallinity of Examples 1-7 and Comparative examples C1 - C3 were measured prior to the annealing (heat treating) step using a TA Instruments Q200 Differential Scanning Calorimeter (obtained from TA Instruments, New Castle, DE). Approximately 10 mg of each film was heated from 0°C to 220°C at 10°C /min. The representative initial crystalline enthalpy was taken as the difference between the cold crystallization and melting enthalpies. The degree of crystallinity was determined using 100 Joules/g as the reference point for a 100% crystalline PLA. Results are shown in Tables 1 and 2 below.

**Table 1**

| Example | Crystallinity (wt-%) | Initial Roughness (Ra - microns) | Annealed Roughness (Ra - microns) |
|---|---|---|---|
| 1 | 19.6 | 3.5 | 3.4 |
| 2 | 1.5 | 2.1 | 3.0 |
| C1 | 0.5 | 0.4 | 0.5 |
| 3 | 36.1 | 3.8 | 3.8 |
| 4 | 29.6 | 4.4 | 4.5 |
| 5 | 2.9 | 4.2 | 4.4 |
| C2 | 1.1 | 3.3 | 0.3 |

**Table 2**

| Example | Crystallinity (wt-%) | Initial Stem Height (microns) | Annealed Stem Height (microns) |
|---|---|---|---|
| 6 | 29.9 | 396 | 381 |
| 7 | 26.2 | 193 | 185 |
| C3 | 5.2 | 86 | 81 |

Various modifications and alterations to this invention will become apparent to those skilled in the art without departing from the scope of this invention. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. A process for forming a film, the process comprising:
applying a molten composition comprising polylactide to a tool roll having a structured surface, wherein the tool roll is at a temperature above the Tg and below the Tm of the polylactide-containing composition;
allowing the molten composition to remain in contact with the tool roll for a time sufficient to convert at least a portion of the polylactide to crystalline polylactide; and
removing a film comprising crystalline polylactide from the tool roll;
wherein the film is continuous and has an embossed, structured surface comprising structure(s) in the form of a negative imprint of the tool roll structured surface;
and further wherein the structure(s) of the embossed, structured surface are retained upon heating the film at a temperature of up to 130°C.

2. The process of claim 1 wherein the film comprises polylactide having at least 1 wt-% crystallinity.

3. The process of claim 1 or claim 2 wherein the film comprises polylactide having no greater than 40 wt-% crystallinity.

4. The process of any one of claims 1 through 3 wherein the tool roll structured surface comprises a plurality of mold cavities and the molten composition is applied to the tool roll under conditions effective to fill the mold cavities.

5. The process of claim 4 wherein mold cavities in the tool roll structured surface form upstanding hook stems on the surface of the film.

6. The process of claim 5 further comprising forming a headed stem mechanical fastener out of the upstanding hook stems on the surface of the film.

7. The process of any one of claims 1 through 3 wherein the tool roll structured surface is textured, and the molten composition is applied to the tool roll under conditions effective to transfer the texture of the structured tool roll surface to the film and provide a matte finish.

8. The process of claim 7 wherein the structures of the film surface have an Ra of at least 1.25 microns.

9. The process of any one of claims 1 through 8 wherein the polylactide-containing composition comprises a plasticizer.

10. The process of claim 8 wherein the polylactide-containing composition comprises a plasticizer that lowers the Tg of the polylactide-containing composition by greater than 5°C.

11. The process of any one of claims 1 through 10 wherein forming a film comprising crystalline polylactide is done in one step.

12. A film comprising polylactide having at least 1 wt-% crystallinity, wherein the film is continuous and comprises an embossed, structured surface comprising structure(s) in the form of a negative imprint of a tool roll structured surface, wherein the structure(s) are retained upon heating the film at a temperature of up to 130°C.

13. The film of claim 12 wherein the polylactide comprises no greater than 40 wt-% crystallinity.

14. A tape comprising a film having at least one surface with a layer of adhesive disposed thereon, wherein the film comprises polylactide having at least 1 wt-% crystallinity, and wherein the film is continuous and comprises an embossed, structured surface comprising structure(s) in the form of a negative imprint of a tool roll structured surface, wherein the structure(s) are retained upon heating the film at a temperature of up to 130°C, and further wherein the structured surface comprises a matte finish.

15. A hook and loop fastener comprising a film comprising polylactide having at least 1 wit-% crystallinity, wherein the film is continuous and comprises an embossed, structured surface comprising structure(s) in the form of a negative imprint of a tool roll structured surface, wherein the structure(s) are retained upon heating the film at a temperature of up to 130°C, and further wherein the structured surface comprises a plurality of hooks.

## Patentansprüche

1. Verfahren zum Bilden einer Folie, wobei das Verfahren Folgendes umfasst:
Auftragen einer geschmolzenen Zusammensetzung, die Polylactid umfasst, auf eine Werkzeugwalze, die eine strukturierte Oberfläche hat, wobei die Werkzeugwalze eine Temperatur oberhalb von Tg und unterhalb des Tm der polylactidhaltigen Zusammensetzung hat;
Ermöglichen, dass die geschmolzene Zusammensetzung in Kontakt mit der Werkzeugwalze über eine Zeit bleibt, die zum Konvertieren zumindest eines Teils des Polylactids in kristallines Polylactid ausreichend ist; und
Entfernen einer Folie, die kristallines Polylactid umfasst, von der Werkzeugwalze;
wobei die Folie durchgehend ist und eine geprägte, strukturierte Oberfläche hat, die Struktur(en) in Form eines Negativabdrucks der strukturierten Werkzeugwalzenoberfläche umfasst;
und ferner, wobei die Struktur(en) der geprägten, strukturierten Oberfläche beim Erwärmen der Folie bei einer Temperatur von bis zu 130 °C beibehalten wird/werden.

2. Verfahren nach Anspruch 1, wobei die Folie Polylactid umfasst, welches mindestens 1 Gew-% Kristallinität hat.

3. Verfahren nach Anspruch 1 oder 2, wobei die Folie Polylactid umfasst, welches eine Kristallinität von nicht mehr als 40 Gew-% hat.

4. Verfahren nach einem der Ansprüche 1 bis 3, wobei die strukturierte Werkzeugwalzenoberfläche mehrere Formhohlräume umfasst und die geschmolzene Zusammensetzung auf die Werkzeugwalze unter Bedingungen aufgetragen wird, die zum Füllen der Formhohlräume wirksam sind.

5. Verfahren nach Anspruch 4, wobei die Formhohlräume in der strukturierten Werkzeugwalzenoberfläche aufrechte Hakenschäfte auf der Oberfläche der Folie bilden.

6. Verfahren nach Anspruch 5, das ferner das Bilden eines mechanischen Befestigungselementes mit Kopf und Schaft aus den aufrechten Hakenschäften auf der Oberfläche der Folie umfasst.

7. Verfahren nach einem der Ansprüche 1 bis 3, wobei die strukturierte Werkzeugwalzenoberfläche texturiert ist und die geschmolzene Zusammensetzung auf die Werkzeugwalze unter Bedingungen aufgetragen wird, die für die Übertragung der Textur der strukturierten Werkzeugwalzenoberfläche auf die Folie effektiv sind und für eine Mattierung sorgen.

8. Verfahren nach Anspruch 7, wobei die Strukturen der Folienoberfläche ein Ra von mindestens 1,25 Mikrometern haben.

9. Verfahren nach einem der Ansprüche 1 bis 8, wobei die polylactidhaltige Zusammensetzung einen Weichmacher umfasst.

10. Verfahren nach Anspruch 8, wobei die polylactidhaltige Zusammensetzung einen Weichmacher enthält, der die Tg der polylactidhaltigen Zusammensetzung um mehr als 5 °C absenkt.

11. Verfahren nach einem der Ansprüche 1 bis 10, wobei das Bilden einer Folie, die kristallines Polylactid umfasst, in einem Schritt erfolgt.

12. Folie, die Polylactid umfasst, welches eine Kristallinität von mindestens 1 Gew-% hat, wobei die Folie durchgehend ist und eine geprägte, strukturierte Oberfläche umfasst, die Struktur(en) in Form von einem Negativabdruck einer strukturierten Werkzeugwalzenoberfläche umfasst, wobei die Struktur(en) beim Erwärmen der Folie bei einer Temperatur von bis zu 130 °C erhalten wird/werden.

13. Film nach Anspruch 12, wobei das Polylactid eine Kristallinität von nicht mehr als 40 Gew-% umfasst.

14. Band, das eine Folie umfasst, die mindestens eine Oberfläche mit einer Schicht von Klebstoff hat, die darauf angeordnet ist, wobei die Folie Polylactid umfasst, das eine Kristallinität von mindestens 1 Gew-% hat, und wobei die Folie durchgängig ist und eine geprägte, strukturierte Oberfläche umfasst, die Struktur(en) in Form eines Negativabdrucks einer strukturierten Werkzeugwalzenoberfläche umfasst, wobei die Struktur(en) beim Erwärmen der Folie bei einer Temperatur von bis zu 130 °C beibehalten wird/werden, und wobei ferner die strukturierte Oberfläche eine Mattierung umfasst.

15. Haken- und Ösenbefestigungsmittel, das eine Folie umfasst, die Polylactid umfasst, welches eine Kristallinität von mindestens 1 Gew-% hat, wobei die Folie durchgehend ist und eine geprägte, strukturierte Oberfläche umfasst, die Struktur(en) in Form von einem Negativabdruck einer strukturierten Werkzeugwalzenoberfläche umfasst, wobei die Struktur(en) beim Erwärmen der Folie bei einer Temperatur von bis zu 130 °C beibehalten wird/werden und wobei ferner die strukturierte Oberfläche mehrere Haken umfasst.

## Revendications

1. Procédé de formation d'un film, le procédé consistant à :
appliquer une composition fondue comprenant un polylactide sur un rouleau outil ayant une surface structurée, le rouleau outil étant à une température supérieure à la température de transition vitreuse Tg et inférieure au point de fusion Tm de la composition contenant le polylactide,
laisser la composition fondue en contact avec le rouleau outil pendant suffisamment de temps pour convertir au moins une partie du polylactide en polylactide cristallin ; et
retirer un film comprenant un polylactide cristallin du rouleau outil ;
dans lequel le film est continu et a une surface structurée, gaufrée, comprenant une ou plusieurs structures sous la forme d'une empreinte négative de la surface structurée du rouleau outil ;
et en outre dans lequel la ou les structures de la surface structurée, gaufrée, sont conservées quand le film est chauffé jusqu'à une température de 130 °C.

2. Procédé selon la revendication 1, dans lequel le film comprend un polylactide ayant une cristallinité d'au moins 1 % en poids.

3. Procédé selon la revendication 1 ou la revendication 2, dans lequel le film comprend un polylactide ayant une cristallinité ne dépassant pas 40 % en poids.

4. Procédé selon l'une quelconque des revendications 1 à 3, dans lequel la surface structurée du rouleau outil comprend une pluralité de cavités de moulage et la composition fondue est appliquée sur le rouleau outil dans des conditions efficaces pour remplir les cavités de moulage.

5. Procédé selon la revendication 4, dans lequel les cavités de moulage dans la surface structurée du rouleau outil forment des tiges de crochets dressées sur la surface du film.

6. Procédé selon la revendication 5, consistant en outre à former une fixation mécanique comportant des tiges pourvues de têtes à partir des tiges de crochets dressées présentes sur la surface du film.

7. Procédé selon l'une quelconque des revendications 1 à 3, dans lequel la surface structurée du rouleau outil est texturée, et la composition fondue est appliquée sur le rouleau outil dans des conditions efficaces pour transférer la texture de la surface structurée du rouleau outil au film et produire un fini mat.

8. Procédé selon la revendication 7, dans lequel les structures de la surface du film ont une valeur Ra d'au moins 1,25 micron.

9. Procédé selon l'une quelconque des revendications 1 à 8, dans lequel la composition contenant le polylactide comprend un plastifiant.

10. Procédé selon la revendication 8, dans lequel la composition contenant le polylactide comprend un plastifiant qui abaisse la Tg de la composition contenant le polylactide de plus de 5 °C.

11. Procédé selon l'une quelconque des revendications 1 à 10, dans lequel la formation d'un film comprenant un polylactide cristallin est réalisée en une seule étape.

12. Film comprenant un polylactide ayant une cristallinité d'au moins 1 % en poids, le film étant continu et comportant une surface structurée, gaufrée, comprenant une ou plusieurs structures sous la forme d'une empreinte négative d'une surface structurée d'un rouleau outil, la ou les structures étant conservées quand le film est chauffé jusqu'à une température de 130 °C.

13. Film selon la revendication 12, dans lequel le polylactide a une cristallinité ne dépassant pas 40 % en poids.

14. Ruban comprenant un film ayant au moins une surface sur laquelle est disposée une couche d'adhésif, le film comprenant un polylactide ayant une cristallinité d'au moins 1 % en poids, le film étant continu et comportant une surface structurée, gaufrée, comprenant une ou plusieurs structures sous la forme d'une empreinte négative d'une surface structurée d'un rouleau outil, la ou les structures étant conservées quand le film est chauffé jusqu'à une température de 130 °C, et la surface structurée ayant en outre un fini mat.

15. Fixation à crochets et à boucles comportant un film comprenant un polylactide ayant une cristallinité d'au moins 1 % en poids, le film étant continu et comportant une surface structurée, gaufrée, comprenant une ou plusieurs structures sous la forme d'une empreinte négative d'une surface structurée d'un rouleau outil, la ou les structures étant conservées quand le film est chauffé jusqu'à une température de 130 °C, et la surface structurée comprenant en outre une pluralité de crochets.
